# FASCICULE DE BREVET EUROPEEN

(11) **EP 0 539 486 B1**
(45) Date de publication et mention de la délivrance du brevet: **28.09.1994**
(21) Numéro de dépôt: 91913610.1
(22) Date de dépôt: 18.07.1991
(51) Int. Cl.: C07D 498/14, C07K 5/06, C07K 7/06

(54) **PROCEDE DE PREPARATION DE DERIVES DE STREPTOGRAMINES**
VERFAHREN ZUR HERSTELLUNG VON STREPTOGRAMINDERIVATEN
PROCESS FOR THE PREPARATION OF STREPTOGRAMINE DERIVATIVES

(30) Priorité: 19.07.1990 FR 9009235
(43) Date de publication de la demande: 05.05.1993
(73) Titulaire: RHONE-POULENC RORER S.A., 92160 Antony (FR)
(72) Inventeur: BARRIERE, Jean-Claude, F-91300 Massy (FR); DUBROEUCQ, Marie-Christine, F-95880 Enghien-les-Bains (FR); FLEURY, Maurice, F-92200 Neuilly-sur-Seine (FR); LARGERON, Martine, F-92200 Neuilly-sur-Seine (FR); PARIS, Jean-Marc, F-77360 Vaires-sur-Marne (FR)
(74) Mandataire: Savina, Jacques
(86) Numéro de dépôt international: FR9100590
(87) Numéro de publication internationale: WO9201691

(56) Documents cités:
- EP-A- 0 133 097
- EP-A- 0 248 703
- FR-A- 1 505 434
- GB-A- 2 206 879
- J. Am. Chem. Soc. (1959), Vol.82, pp.4414-4422
- Bull. Soc. Chim. Belg. (1991), Vol.100 n 9, pp.653-663
- Liebigs Annalen der Chemie, no.1, 1986,, VCH Verlagsgesellschaft mbH(Weinheim, DE) H. Kessler et al. :"Synthese von Virginiamycin S1 und Virginiamycin S4", p. 21-31
- Bulletin de la Société Chimique de France, no. 2, 1968,(Paris, FR )J, Preud'homme et al. :"Pristinamycine. Isolement, caractérisation et identification des constituants", p- 585-591

## Description

Les streptogramines sont des produits connus et mentionnés notamment par J. Preud'homme et coll., Bull. Soc. Chim. Fr., 2, 585-91 (1968) ou par C. Cocito, Antibiotics, 296 (1983).

La synthèse totale de la virginiamycine S a été décrite dans Liebigs Ann. Chem., 21-31 (1986).

Dans les brevets US 4 618 599 et US 4 798 827 ont été décrits des dérivés solubles de la pristinamycine I_{A} et de la virginiamycine S.

La présente invention concerne la préparation de dérivés de streptogramines de formule générale : 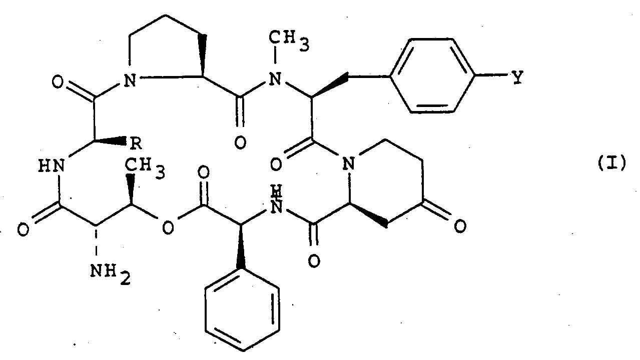
leurs sels, leur préparation et leur utilisation.

Dans la formule générale (I), le symbole R représente un radical méthyle ou éthyle et le symbole Y représente un atome d'hydrogène ou un radical méthylamino, diméthylamino ou un radical de structure : 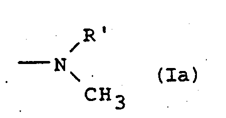
dans laquelle R' est un radical protecteur d'amino.

A titre d'exemple, R' peut être avantageusement choisi parmi, trifluoroacétyle, benzyloxycarbonyle, propène-2 yloxycarbonyle, nitrobenzyloxycarbonyle, fluorènyl-9 méthyloxycarbonyle ou o.nitrobenzyloxyacétyle.

Selon l'invention les dérivés de streptogramines de formule générale (I) peuvent être obtenus par coupure réductive d'une streptogramine de formule générale : 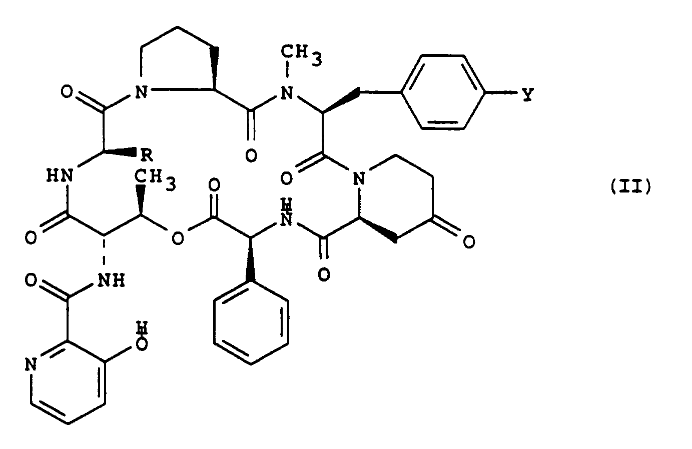
dans laquelle Y et R sont définis comme ci-dessus.

La coupure réductive est mise en oeuvre par traitement en milieu acide, en présence d'un métal réducteur.

La réaction s'effectue en milieu acide fort, à un pH inférieur à 2, en présence d'un métal réducteur dont le potentiel d'oxydo-réduction est inférieur à -0,94V (e.c.s.). On opère en milieu aqueux, ou en milieu hydroalcoolique (par exemple dans un mélange eau-méthanol ou eau-éthanol), à une température comprise entre -10 et 60°C. L'acide peut être choisi parmi les acides sulfurique, chlorhydrique, bromhydrique, trifluoracétique ou méthanesulfonique.

A titre d'exemple, le métal réducteur peut être avantageusement choisi parmi le zinc, le magnésium, l'aluminium ou l'amalgame de sodium. De préférence on opère sous azote.

Selon l'invention, les dérivés de la streptogramine de formule générale (I) peuvent également être préparés par réduction électrochimique en milieu acide d'un dérivé de streptogramine de formule générale (II).

L'électrolyse à potentiel contrôlé est réalisée en solution acide aqueuse ou hydroalcoolique contenant jusqu'à 50% d'alcool (méthanol ou éthanol par exemple) à une température comprise entre 0 et 60°C, sous agitation constante et sous atmosphère d'azote, dans une cellule d'électrolyse dans laquelle la cathode est constituée d'une nappe de mercure. Le potentiel de l'électrode de travail E est tel que -0,9 > E > -1,1 V (e.c.s).

L'acide est avantageusement choisi parmi les acides chlorhydrique, bromhydrique ou sulfurique.

Les produits obtenus par le procédé selon l'invention sont particulièrement intéressants comme intermédiaires pour la préparation de dérivés de streptogramines biologiquement actifs.

Plus précisément ils servent d'intermédiaires pour obtenir de nouveaux dérivés de streptogramines de formule générale : 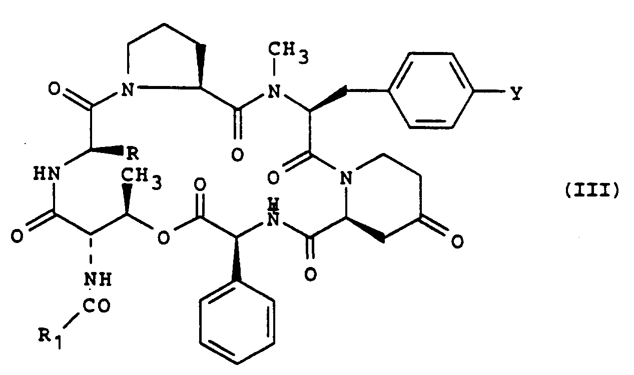
dans laquelle Y et R sont définis comme précédemment et R₁ représente un radical phényle ou pyridyle monosubstitué par un radical alcoyle droit ou ramifié contenant 2 à 6 atomes de carbone ou par un radical trifluorométhyle, ou représente un radical phényle disubstitué par des radicaux alcoyle droits ou ramifiés contenant 1 à 6 atomes de carbone ou nitro, ou représente un radical naphtalènyle ou un radical quinolyle substitué par un atome d'halogène, qui permettent de supprimer la résistance des tumeurs aux substances anticancéreuses, et sont particulièrement intéressants comme agents associés aux traitements des cancers.

Les produits de formule générale (III) peuvent être obtenus par action d'un acide de formule générale :

R₁―COOH (IV)

dans laquelle R₁ est défini comme ci-dessus, ou d'un dérivé réactif de cet acide, sur un produit de formule générale (I).

Lorsque l'on fait réagir un dérivé réactif, ce dernier peut être choisi parmi l'anhydride, un anhydride mixte, un halogénure d'acide ou un ester réactif.

La réaction s'effectue en milieu organique, éventuellement en présence d'un accepteur d'acide tel qu'une base organique azotée (une trialcoylamine, une pyridine, la N-méthylmorpholine, le diaza-1,8 bicyclo[5.4.0]undécène-7, le diaza-1,5 bicyclo[4.3.0]nonène-5 par exemple) dans un solvant organique tel qu'un solvant chloré (chlorure de méthylène, dichloréthane, chloroforme par exemple) , un amide (diméthylformamide par exemple), un oxyde (diméthylsulfoxyde par exemple), une cétone (acétone par exemple) ou un éther (tétrahydrofuranne par exemple), à une température comprise entre -20 et 60°C. Il est également possible d'opérer en présence d'un agent de condensation tel qu'un carbodiimide (le dicyclohexylcarbodiimide, le chlorhydrate d'éthyl-1 (diméthylamino-3 propyl)-3 carbodiimide par exemple) et éventuellement en présence d'un catalyseur tel que l'hydroxybenzotriazole, dans un solvant cité ci-dessus, ou en présence d'un carbonate ou d'un bicarbonate de métal alcalin ou alcalino-terreux à la température définie précédemment.

Les produits de formule générale (I) ou les produits de formule générale (III) peuvent être purifiés par les méthodes connues telles que la cristallisation ou la chromatographie.

Les produits obtenus selon l'invention peuvent être transformés en sels d'addition avec les acides selon les méthodes habituelles. A titre d'exemple les sels peuvent être des sels d'addition avec des acides minéraux tels que chlorhydrates, bromhydrates, sulfates, nitrates, phosphates, ou avec des acides organiques tels que les acétates, propionates, succinates, maléates, fumarates, méthanesulfonates, tartrate, camphosulfonate ou des dérivés de substitution de ces composés.

Les produits de formule générale (III) préparés à partir des produits selon l'invention sont des agents capables de maintenir la chimiosensibilité des tumeurs, ou de restaurer la chimiosensibilité des tumeurs devenues résistantes.

Leur activité a été mise en évidence sur une lignée cellulaire P388 resistante à la doxorubicine (P388/DOX) [R.K. JOHNSON et coll., Canc. Treat. Rep., 62, 1535-1547 (1978)].

Au jour 0, des tubes sont ensemencés avec 3,6 cm³ d'une suspension de cellules P388/DOX (2 x 10⁵ cellules/cm³ dans du milieu RPMI 1640 contenant 10% de sérum de veau foetal). Les tubes sont incubés avec les produits à tester, à différentes concentrations et à 37°C (3 tubes par concentration); les produits sont solubilisés dans du milieu complet et additionnés sous le volume final de 0,4 cm³. Une autre série de tubes est également incubée avec les produits à tester à différentes concentrations, mais en présence de 1 µg/cm³ de doxorubicine.

Au jour 4, les cellules sont comptées. Les résultats sont exprimés par la CI₅₀ (µM). La CI₅₀ correspond à la concentration de produit permettant d'obtenir 50% de cytotoxicité due à la doxorubicine, c'est à dire à une concentration de produit non cytotoxique par lui-même.

Dans cette technique, les produits de formule générale (III) se sont montrés actifs à des concentrations comprises entre 0,2 et 2 µM.

De plus les dérivés de streptogramines sont faiblement toxiques : ils se sont généralement montrés atoxiques à des doses de 200 mg/kg par voie sous cutanée chez la souris.

Les exemples suivants donnés à titre non limitatif illustrent la présente invention.

Dans les exemples qui suivent, sauf mention spéciale, les spectres de RMN ont été faits à 250 MHz dans le deutérochloroforme; les déplacements chimiques sont exprimés en ppm. Les chromatographies "flash" sont effectuées sous une pression moyenne de 50 kPa, en utilisant une silice de granulométrie 40-53 µm, selon W.C. STILL et coll., J. Org. Chem., 43, 2923 (1978).

### Exemple 1

200 g de pristinamycine IA sont ajoutés à 5 litres d'une solution aqueuse 1N d'acide chlorhydrique agitée sous atmosphère d'azote. On obtient une solution trouble dont le pH est voisin de 0,5 et dont la température est de 27°C. On ajoute ensuite en 5 minutes 150 g de zinc en poudre, la température du mélange réactionnel croît jusqu'à 32°C. Le mélange réactionnel est agité 1 heure à une température voisine de 30°C, le pH du mélange est alors voisin de 1. Après addition de 2 litres de dichlorométhane, le pH du mélange réactionnel est ajusté à une valeur voisine de 4 par addition lente de 110 cm³ d'une solution aqueuse 10N d'hydroxyde de sodium. La phase organique est décantée, la phase aqueuse est extraite avec 500 cm³ de dichlorométhane puis les phases organiques réunies sont filtrées sur un lit de Supercel®. Le filtrat est lavé avec 3 fois 100 cm³ d'eau distillée, séché sur du sulfate de sodium, filtré puis concentré jusqu'à un volume de 500 cm³ sous pression réduite (2,7kPa) à une température voisine de 30°C. La solution obtenue est déposée sur une colonne de 7 kg de gel de silice (diamètre : 15 cm, hauteur : 92 cm). La colonne est éluée avec un mélange dichlorométhane/ méthanol (97/3 en volumes) en faisant des fractions de 1,5 litres. On concentre à sec les fractions 7 à 13 sous pression réduite (2,7 kPa) à une température voisine de 30°C, le résidu obtenu est trituré pendant 2 heures dans 400 cm³ de pentane, filtré puis séché sous pression réduite (0,27 kPa) à une température voisine de 20°C. On obtient ainsi 66 g de dès (hydroxy-3 picolinoyl) pristinamycine IA sous forme d'une poudre blanche fondant vers 206°C.

### Spectre de RMN :

| δ (ppm) | Forme | Attribution |
|---|---|---|
| 0,35 | dd | 5 β2 |
| 0,91 | t | 2γ |
| 1,1-1,3 | m | 3 γ2 et 3 β2 |
| 1,38 | d | 1 γ |
| 1,52 | m | 3 β1 |
| 1,65 et 1,8 | 2m | 2 β1 et 2β2 |
| 2,02 | m | 3 β1 |
| 2,1 à 2,35 | m | 5 δ2, 5β1 et 5δ1 |
| 2,74 | dt | 5 ε2 |
| 2,87 | s | N(CH₃)₂ |
| 2,98 | dd | 4 β2 |
| 3,3 | s | NCH₃ |
| 3,2 à 3,4 | m | 3 δ2, 4 β1 et 1α |
| 3,52 | m | 3 δ1 |
| 4,6 à 4,9 | m | 3 α, 5ε1 et 2α |
| 4,97 | d | 5 α |
| 5,24 | dd | 4 α |
| 5,75 | q (large) | 1 β |
| 5,83 | d | 6 α |
| 6,63 | d | 4 ε |
| 7,1 à 7,35 | m | 4 δ et aromatiques |
| 8 | d | 2 NH |
| 8,63 | d | 6 NH |

### Exemple 2

A une solution de 10 g de virginiamycine S1 dans un mélange de 200 cm³ de méthanol et de 50 cm³ d'une solution aqueuse 5N d'acide chlorhydrique, maintenue sous une atmosphère d'azote, on ajoute 5 g de zinc en poudre. La suspension grise obtenue est agitée 1 heure à une température voisine de 20°C. Le pH du mélange réactionnel est alors de 1,2, il est ensuite ajusté à 5 par addition de 100 cm³ d'une solution aqueuse 1N d'hydroxyde de sodium. Le mélange est extrait par 3 fois 300 cm³ de dichlorométhane; les phases organiques réunies sont séchées sur du sulfate de sodium, filtrées, puis concentrées à sec sous pression réduite (2,7 kPa) à une température voisine de 30°C. On obtient ainsi 8,1 g de résidu qui est purifié par chromatographie flash (éluant : dichlorométhane/méthanol 98/2 en volumes) en recueillant des fractions de 80 cm³. Les fractions 10 à 30 sont concentrées à sec sous pression réduite (2,7 kPa) à une température voisine de 30°C. On obtient ainsi 3,9 g de dès (hydroxy-3 picolinoyl) virginiamycine S1 sous forme d'une poudre blanche fondant vers 175°C.

### Spectre de RMN :

| δ (ppm) | Forme | Attribution |
|---|---|---|
| 0,11 | dd | 5 β2 |
| 0,9 | t | 2 γ |
| 1,13 | m | 3 γ2 et 3 β2 |
| 1,35 | d | 1 γ |
| 1,5 | m | 3 γ1 |
| 1,64 et 1,8 | 2m | 2 β1 et 2 β2 |
| 1,99 | m | 3 β1 et 5 δ2 |
| 2,08 | m | 5 β1 |
| 2,22 | m | 5 δ1 |
| 2,72 | dt | 5 ε2 |
| 3,04 | dd | 4 β2 |
| 3,22 | s | 3 δ2 |
| 3,26 | s | NCH₃ |
| 3,3 | m | 1 α |
| 3,38 | m | 4 β1 |
| 3,51 | m | 3 δ1 |
| 1,58 | dd | 3 α |
| 4,65 | m | 5 ε1 |
| 4,75 | dt | 2 α |
| 4,97 | d | 5 α |
| 5,3 | dd | 4 α |
| 5,73 | dq | 1 β |
| 5,83 | d | 6 α |
| 7,05 à 7,3 | m | aromatiques en 4 et 6 |
| 8 | d | 2 NH |
| 8,61 | d | 6 NH |

### Exemple 3

L'électrolyse est réalisée au moyen d'un montage à 3 électrodes.

La cellule d'électrolyse est constituée par un ensemble de verreries rodées, les compartiments anodique et cathodique étant concentriques et séparés par une paroi de verre fritté de porosité 7. Un potentiostat-galvanostat Tacussel PJT 120V-1A et un intégrateur Tacussel IG5 N complètent le circuit.

L'électrode de travail est une nappe de mercure dont l'aire est égale à 60 cm². L'électrode auxiliaire est une lame de platine. L'électrode de référence est une électrode au calomel à solution saturée de chlorure de potassium (e.c.s.).

On procède à l'électrolyse d'une solution de 0,35 g de pristinamycine IA dans 200 cm³ d'acide sulfurique 1N , sous atmosphère d'azote, à 25°C, sur une nappe de mercure dont le potentiel est fixé à -1,0 V e.c.s. A la fin de l'électrolyse, lorsque l'intensité du courant est devenue négligeable (2 mA) devant l'intensité du courant initial (120 mA), le pH de la solution d'électrolyse est amené aux alentours de 6,0 au moyen d'une solution de carbonate de potassium 5M. La solution résultante est alors extraite avec 200 cm³ de dichlorométhane. La phase organique est séchée sur du sulfate de sodium et concentrées à sec sous pression réduite (2,7 kPa), à une température voisine de 30°C. Le résidu est repris par 1 cm³ de dichlorométhane et la solution obtenue est déposée sur une colonne de 15 g de gel de silice (diamètre : 1,5 cm, hauteur : 60 cm). La colonne est éluée avec un mélange dichlorométhane/ méthanol (98/2 en volumes), en recueillant des fractions de 5 cm³. Les fractions 22 à 32 sont concentrées à sec sous pression réduite (2,7 kPa), à une température voisine de 30°C. On obtient 0,15 g de dès (hydroxy-3 picolinoyl) pristinamycine IA dont les caractéristiques sont identiques à celles du produit obtenu à l'exemple 1.

### Exemple 4

En opérant comme décrit précédemment à l'exemple 3, mais à une température de 10°C, on procède à l'électrolyse de 0,35 g de pristinamycine I_{A}. On obtient 0,105 g de dès(hydroxy-3 picolinoyl) pristinamycine IA dont les caractéristiques sont identiques à celles du produit obtenu à l'exemple 1.

### Exemple 5

En opérant comme décrit précédemment à l'exemple 3, mais à une température de 40°C, on procède à l'électrolyse de 0,35 g de pristinamycine I_{A}. On obtient 0,09 g de dès(hydroxy-3 picolinoyl) pristinamycine IA dont les caractéristiques sont identiques à celles du produit obtenu à l'exemple 1.

### Exemple 6

En opérant comme décrit précédemment à l'exemple 3, mais sur une nappe de mercure dont le potentiel est fixé à -0,9 V e.c.s., on procède à l'électrolyse de 0,35 g de pristinamycine I_{A} dans 200 cm³ d'acide chlorhydrique 1N. On obtient 0,09 g de dès(hydroxy-3 picolinoyl) pristinamycine IA dont les caractéristiques sont identiques à celles du produit obtenu à l'exemple 1.

### Exemple 7

La dés(hydroxy-3 picolinoyl) pristinamycine I_{C} peut être obtenue comme décrit à l'exemple 1, mais à partir de 1 g pristinamycine I_{C}, de 25 cm³ d'une solution aqueuse 1N d'acide chlorhydrique et de 0,5g de zinc en poudre.

On obtient ainsi 0,8 g d'un résidu qui est purifié par chromatographie flash (éluant: chlorure de méthylène, méthanol 98/2 en volumes) en recueillant des fractions de 10 cm³. Les fractions 19 à 40 sont réunies puis concentrées à sec sous pression réduite (2,7 kPa) à 30°C. On obtient ainsi 0,3 g de dés(hydroxy-3 picolinoyl) pristinamycine I_{C} sous forme d'une poudre blanche.

### Spectre de RMN

| | | |
|---|---|---|
| 1,34 | d | 2β |
| 7,1 à 7,3 | mt | aromatiques et 4δ |
| 8,1 | d | >NH en 2 |
| 8,65 | d | >NH en 6 |

### Exemple 8

La dés(hydroxy-3 picolinoyl) pristinamycine I_{B} peut être obtenue comme décrit à l'exemple 1 mais à partir de 1 g de pristinamycine I_{B}, 25 cm³ d'une solution aqueuse 1N d'acide chlorhydrique et de 0,5 g de zinc en poudre. On obtient ainsi, 0,8 g d'un résidu qui est purifié par chromatographie flash (éluant : chlorure de méthylène, méthanol 98/2 en volumes) en recueillant des fractions de 10 cm³. Les fractions 18 à 36 sont réunies et concentrées à sec sous pression réduite (2,7 kPa) à 30°C. On obtient ainsi 0,2 g de dés(hydroxy-3 picolinoyl) pristinamycine I_{B} sous forme d'une poudre blanche.

### Spectre de RMN

| | | |
|---|---|---|
| 1,6 à 1,9 | mt | 2β1 et 2β2 |
| 1,9 à 2,3 | mt | 5β1, 5γ1, 5γ2, 3β1 et NH₂ |
| 2,74 | s et mt | ArNHCH₃ et 5ε₂ |

### Exemple 9

La dés(hydroxy-3 picolinoyl) trifluoroacétyl-4N pristinamycine I_{B} peut être obtenue comme décrit à l'exemple 2 mais à partir de 0,68 g de trifluoroacétyl-4N pristinamycine I_{B}, 2,8 cm³ d'une solution aqueuse 5N d'acide chlorhydrique dans 14 cm³ de méthanol et de 0,34 g de zinc en poudre dans 14 cm³ de méthanol.

On obtient 0,52 g d'un résidu qui est purifié par chromatographie flash (éluant : chlorure de méthylène,méthanol 97/3 en volumes) en recueillant des fractions de 5 cm³ en volumes. Les fractions 6 à 16 sont réunies et concentrées à sec sous pression réduite (2,7 kPa) à 30°C. On obtient ainsi 0,19 g de des (hydroxy-3 picolinoyl) trifluoroacétyl-4N pristinamycine I_{B} sous forme d'une poudre blanche.
Spectre de masse réalisé sur un appareil VG Autospec en FAB canon Cs dans une matrice NBA :

(M+H)⁺ = 828

(M-H₂O+H)⁺ = 810

### Spectre de RMN

| | |
|---|---|
| 1,55 à 1,85 mt | 3γ1, 2β1 et 2β2 |
| 3,23 et 3,3 2s | >N-CH₃ en 4 [ArN(CH₃)COCF₃+-CO-N(CH₃)-] |

La trifluoroacétyl-4N pristinamycine I_{B} est obtenue de la manière suivante :
A 25 mg de pristinamycine I_{B} en solution dans 1 cm³ de dichlorométhane anhydre on ajoute, 6 mg d'anhydride trifluoroacétique. On laisse agiter 12 heures. On ajoute à nouveau 6 mg d'anhydride trifluoracétique. Le mélange est agité 4 heures au reflux puis refroidi et ajusté à pH 7-8 avec une solution aqueuse de bicarbonate de sodium. La phase organique est décantée, la phase aqueuse lavée par 2 fois 2 cm³ d'eau. Les phases organiques sont réunies, séchées sur sulfate de sodium, filtrées puis évaporées à sec sous pression réduite (2,7 kPa) à 30°C. On obtient ainsi 25 mg de trifluoroacétyl-4N pristinamycine I_{B} sous forme d'une poudre blanche.

Les produits selon l'invention peuvent être utilisés de la manière suivante :

### Exemple d'utilisation 1

A une solution de 1,5 g de dès(hydroxy-3 picolinoyl) pristinamycine IA dans 25 cm³ de chlorure de méthylène maintenue à 5°C, on ajoute simultanément, goutte à goutte, 0,43 cm³ de chlorure de tertiobutyl-4 benzoyle en solution dans 5 cm³ de chlorure de méthylène et 0,34 cm³ de triéthylamine. Le mélange réactionnel est ensuite agité 2 heures à une température voisine de 20°C, puis on ajoute 20 cm³ d'eau distillée. La phase organique est décantée, la phase aqueuse est extraite 2 fois par 25 cm³ de chlorure de méthylène, les phases organiques réunies sont lavées par 20 cm³ d'eau distillée puis séchées sur du sulfate de magnésium. Après filtration puis concentration à sec sous pression réduite (2,7 kPa) à 30°C des phases organiques, on obtient 2,2 g d'un résidu qui est purifié par chromatographie flash (éluant : acétate d'éthyle) en recueillant des fractions de 10 cm³. Les fractions 8 à 11 sont réunies et concentrées à sec sous pression réduite (2,7 kPa) à 30°C. On obtient ainsi 1,65 g de (tertiobutyl-4 benzoyl)-1 dès(hydroxy-3 picolinoyl) pristinamycine IA sous forme d'une poudre blanche fondant vers 206°C.

### Spectre RMN :

0,24 ( dd, 5 β2)
1,34 ( s, C(CH₃)₃)
2 à 2,3 ( m, 5 δ2, 5 β1 et 5 δ1)
noyau benzènique en 1 :
7,57 ( d, H3 et H5 )
7,88 ( d, H2 et H6)
Le chlorure de tertiobutyl-4 benzoyle peut être préparé selon la méthode décrite par F. Bell et R.D. Wilson , J. Chem. Soc. 2340 (1956).

### Exemple d'utilisation 2

A une solution de 1,5 g de dès(hydroxy-3 picolinoyl) pristinamycine IA, de 0,42 g d'acide dinitro-3,4 benzoïque et de 0,027 g d'hydroxy-1 benzotriazole dans 35 cm³ de chlorure de méthylène maintenue à 5°C, on ajoute, goutte à goutte, 0,42 g de chlorhydate d'éthyl-1 (diméthylamino-3 propyl)-3 carbodiimide en solution dans 25 cm³ de chlorure de méthylène. Le mélange réactionnel est ensuite agité 2 heures à une température voisine de 5°C, puis 2 heures à une température voisine de 20°C. On ajoute 20 cm³ d'eau - distillée;
La phase organique est décantée, la phase aqueuse est extraite 2 fois par 20 cm³ de chlorure de méthylène, les phases organiques réunies sont lavées par 20 cm³ d'eau distillée puis séchées sur du sulfate de sodium. Après filtration puis concentration à sec sous pression réduite (2,7 kPa) à 30°C des phases organiques, on obtient 2,07 g d'un résidu qui est purifié par chromatographie flash (éluant : acétate d'éthyle) en recueillant des fractions de 10 cm³. Les fractions 61 à 131 sont réunies et concentrées à sec sous pression réduite (2,7 kPa) à 30°C. On obtient ainsi 1,25 g de (dinitro-3,4 benzoyl)-1 dès (hydroxy-3 picolinoyl) pristinamycine IA sous forme d'une poudre blanche fondant vers 212°C.

### Spectre de RMN :

0,25 ( dd, 5 β2)
2 à 2,3 ( m, 5 δ2, 5 β1 et 5 δ1)
noyau benzènique en 1 :
8,05 ( d, H5 )
8,38 ( dd, H6 )
8,65 ( d, H2 )

### Exemple d'utilisation 3

A une solution de 0,394 g d'acide butyl-5 picolinique et de 0,22 g de N-méthyl morpholine dans 20 cm³ de dichlorométhane maintenue à -10°C, on ajoute 0,285 cm³ de chloroformiate d'isobutyle, puis on agite le mélange réactionnel 1 heure à -10°C. On additionne ensuite, à -5°C, une solution de 1,5 g de dès(hydroxy-3 picolinoyl) pristinamycine IA dans 15 cm³ de dichlorométhane. Le mélange obtenu est agité pendant 16 heures à une température voisine de 20°C, puis on ajoute 50 cm³ d'eau distillée. La phase organique est décantée et lavée avec 50 cm³ d'une solution aqueuse saturée de chlorure de sodium. Après séchage sur du sulfate de magnésium, filtration puis concentration à sec sous pression réduite (2,7 kPa) à une température voisine de 30°C on obtient 1,8 g de résidu qui est purifié par chromatographie flash (éluant : dichloroéthane-1,2/ méthanol 97/3 en volumes) en recueillant des fractions de 60 cm³^{.} Le résidu obtenu après concentration à sec sous pression réduite (2,7 kPa) à une température voisine de 30°C des fractions 12 à 16 est trituré dans un mélange de 50 cm³ d'éther éthylique et de 100 cm³ de pentane. Le solide est filtré et séché à une température voisine de 20°C, on obtient ainsi 0,75 g de (butyl-5 picolinoyl)-1 dès(hydroxy-3 picolinoyl) pristinamycine IA sous forme d'une poudre blanche fondant à 165°C.

### Spectre RMN(400 Hz) :

0,7 ( dd, 5 β2)
1,04 ( t, ·CH₃ de la chaine bùtyle )
1,47 ( m, CH₃CH₂ de la chaine butyle)
1,74 ( m, CH₃CH₂CH₂ de la chaine butyle et 2 β1)
2,4 ( d, 5 β1)
2,75 à 2,9 ( m, CH₃CH₂CH₂CH₂ de la chaine butyle et 5 ε2)
noyau pyridinique en 1 :
7,8 ( dd, H4 )
8,23 ( d, H3 )
8,28 ( d, H6 )

### Exemple d'utilisation 4

En opérant d'une manière analogue à celle décrite à l'exemple d'utilisation 1, mais à partir de 1,5 g dès(hydroxy-3 picolinoyl) pristinamycine IA et de 0,37 g de chlorure de diméthyl-2,4 benzoyle et après purification par chromatographie flash (éluant : dichloroéthane-1,2/ méthanol 97,5/2,5 en volumes) en recueillant des fractions de 30 cm³ et concentration à sec sous pression réduite (2,7 kPa) des fractions 15 à 19 à une température voisine de 30°C, on obtient 0,99 g de (diméthyl-2,4 benzoyl)-1 dès(hydroxy-3 picolinoyl) pristinamycine IA sous forme d'une poudre blanche fondant vers 170°C.

### Spectre RMN :

0,21 ( dd, 5 β2 )
2,1 ( d, 5 β1 )
2,4 et 2,56 ( 2s, les CH₃ du noyau benzènique en 1 )
noyau benzènique en 1 :
7,11 ( d(large), H5 )
7,20 ( s(large), H3 )
7,48 ( d, H6 )

### Exemple d'utilisation 5

En opérant d'une manière analogue à celle décrite à l'exemple d'utilisation 1, mais à partir de 1,5 g dès (hydroxy-3 picolinoyl) pristinamycine IA et de 0,33 cm³ de chlorure de trifluorométhyl-4 benzoyle et après purification par chromatographie flash (éluant : dichlorométhane/ éthanol 98/2 en volumes) en recueillant des fractions de 20 cm³ et concentration à sec sous pression réduite (2,7 kPa) des fractions 10 à 15 à une température voisine de 30°C, on obtient 0,69 g de (trifluorométhyl-4 benzoyl)-1 dès(hydroxy-3 picolinoyl) pristi-namycine IA sous forme d'une poudre blanche fondant vers 178°C.

### Spectre RMN(400 MHz):

0,25 ( dd, 5 β2)
2,05 à 2,3 ( m, 5 δ2, 5 β1 et 5 δ1)
noyau benzènique en 1 :
7,79 ( d, H3 et H5 )
8,03 ( d, H2 et H6 )

### Exemple d'utilisation 6

En opérant d'une manière analogue à celle décrite à l'exemple d'utilisation 1, mais à partir de 1,5 g dès(hydroxy-3 picolinoyl) pristinamycine IA, de 0,63 cm³ de triéthylamine et de 0,91 g de chlorure de l'acide chloro-2 quinoléinecarboxylique-4 et après purification par chromatographie flash (éluant : dichlorométhane/méthanol 98/2 en volumes) en recueillant des fractions de 30 cm³ et concentration à sec sous pression réduite (2,7 kPa) des fractions 10 à 15 à une température voisine de 30°C, on obtient 1,02 g de (chloro-2 quinolyl-4)carbonyl-1 dès(hydroxy-3 picolinoyl)pristinamycine IA sous forme d'une poudre blanche fondant à une température supérieure à 260°C.

Le chlorure de l'acide chloro-2 quinoléinecarboxylique-4 peut être préparé selon la méthode décrite par B. Mulert, Chem. Ber., 39, 1901 (1906).

### Spectre de RMN(400 MHz) :

-0,1 ( dd, 5 β2)
1,97 ( d, 5 β1 )
noyau quinoléine :
7,91 ( s, H3 )
7,78, 7,95, 8,18, 8,30 ( 2t, 2d, H₅, H₆, H₇ et H8 )

### Exemple d'utilisation 7

En opérant d'une manière analogue à celle décrite à l'exemple d'utilisation 1, mais à partir de 1,5 g dès(hydroxy-3 picolinoyl) pristinamycine IA et de 0,42 g de chlorure de naphtoyle-1 et après purification par chromatographie flash (éluant : acétate d'éthyle/méthanol 96/4 en volumes) en recueillant des fractions de 25 cm³ et concentration à secs sous pression réduite (2,7 kPa) des fractions 12 à 20 à une température voisine de 30°C, on obtient 1,06 g de (naphtoyl-1)-1 dès (hydroxy-3 picolinoyl) pristinamycine IA sous forme d'une poudre verdâtre fondant vers 170°C.

### Spectre-de RMN :

0 ( dd, 5 β2)
1,9 à 2,25 ( m, 3 β1, 5 β1, 5 δ2 et 5 δ1 )
noyau naphtyle :
7,5 à 7,8 ( m, H3, H6, H7 )
7,85 à 8,1 ( m, H4, H5, H8 )
8,46 ( d(large), H2 )

### Exemple d'utilisation 8

A une solution de 0,7 g de dès(hydroxy-3 picolinoyl) virginiamycine S1, de 0,21 g d'acide dinitro-3,4 benzoïque et de 0,014 g d'hydroxy-1 benzotriazole dans 20 cm³ de chlorure de méthylène maintenue à 5°C, on ajoute, goutte à goutte, 0,21 g de chlorhydate d'éthyl-1 (diméthylamino-3 propyl)-3 carbodiimide en solution dans 25 cm³ de chlorure de méthylène. Le mélange réactionnel est ensuite agité 2 heures à une température voisine de 5°C, puis 2 heures à une température voisine de 20°C. On ajoute 20 cm³ d'eau distillée; La phase organique est décantée, la phase aqueuse est extraite 2 fois par 20 cm³ de chlorure de méthylène, les phases organiques réunies sont lavées par 20 cm³ d'eau distillée puis séchées sur du sulfate de sodium. Après filtration puis concentration à sec sous pression réduite (2,7 kPa) à 30°C des phases organiques, on obtient 0,9 g d'un résidu qui est purifié par chromatographie flash (éluant: dichlorométhane/méthanol 97,5/2,5 en volumes) en recueillant des fractions de 8 cm³. Les fractions 36 à 60 sont réunies et concentrées à sec sous pression réduite (2,7 kPa) à 30°C. On obtient ainsi 0,5 g de (dinitro-3,4 benzoyl)-1 dès(hydroxy-3 picolinoyl) virginiamycine S1 sous forme d'une poudre blanche fondant vers 190°C.

### Spectre de RMN :

0,05 ( dd, 5 β2)
1,9 à 2,3 ( m, 3 β, 5 δ2, 5 β1 et 5 δ1)
noyau benzènique en 1 :
8,03 ( d, H5 )
8,41 ( dd, H6 )
8,66 ( d, H2 )

### Exemple d'utilisation 9

En opérant d'une manière analogue à celle décrite à l'exemple d'utilisation 1, mais à partir de 1 g dès(hydroxy-3 picolinoyl) virginiamycine S1, de 0,31 cm³ de triéthylamine et de 0,64 g de chlorure de l'acide chloro-2 quinoléinecarboxylique-4 et après purification par chromatographie flash (éluant : dichlorométhane/méthanol 98/2 en volumes) en recueillant des fractions de 10 cm³ et concentration à sec sous pression réduite (2,7 kPa) des fractions 15 à 35 à une température voisine de 30°C, on obtient 0,85 g de (chloro-2 quinolyl-4)carbonyl-1 dès(hydroxy-3 - picolinoyl) virginiamycine S1 sous forme d'une poudre blanche fondant à une température supérieure à 260°C.

### Spectre de RMN :

-0,4 ( dd, 5 β2)
2 à 2,2 ( m, 5 δ2, 5δ1 et 5 β1 )
noyau quinoléine :
7,8 ( s, H3 )
7,71, 7,93, 8,18, 8,30 (2dt, et d, H5, H6, H7, H8)

### Exemple d'utilisation 10

En opérant comme à l'exemple d'utilisation 2, mais à partir de 0,3 g de dés(hydroxy-3 picolinoyl) pristinamycine I_{C}, de 0,09 g d'acide dinitro-3,4 benzoïque, de 0,006 g d'hydroxy-1 benzotriazole et de 0,09 g de chlorhydrate d'éthyl-1 (diméthylamino-3 propyl)-3 carbodiimide, on obtient 0,3 g d'un résidu qui est purifié par chromatographie flash (éluant : chlorure de méthylène,méthanol 97/3 en volumes) en recueillant des fractions de 5 cm³^{.} Les fractions 31 à 45 sont réunies et concentrées à sec sous pression réduite (2,7 kPa) à 30°C. On obtient ainsi 0,24 g de (dinitro-3,4 benzoyl)-1 dès(hydroxy-3 picolinoyl) pristinamycine I_{C} sous forme d'une poudre blanche fondant vers 200-210°C.

### Spectre de RMN :

1,26 et 1,32 (2d, 1γ et 2β)
7,10 à 7,4 (mt, aromatiques)
8,06 (d, 1'H₅)
8,45 (dd, 1'H₆)
8,63 (d, >NH en 1)
8,66 (d, 1'H₂)
8,85 (d, >NH en 6)

## Revendications

1. Procédé de préparation d'un dérivé de la streptogramine de formule générale : 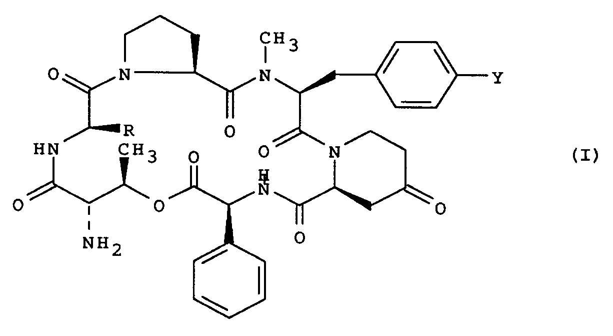 dans laquelle Y est un atome d'hydrogène ou un radical méthylamino ou diméthylamino, ou un radical de structure : 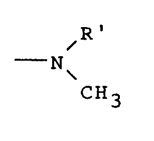 dans laquelle R' est un radical protecteur d'amino et R est un radical méthyle, ou éthyle, caractérisé en ce que l'on effectue la coupure réductive en milieu acide d'un dérivé de streptogramine de formule générale : 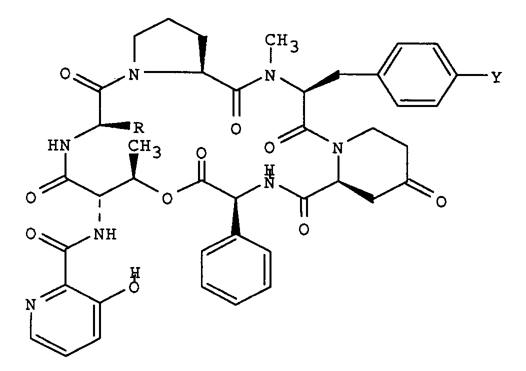 dans laquelle Y et R sont définis comme ci-dessus,
soit par un acide fort à un pH inférieur à 2, en présence d'un métal réducteur dont le potentiel d'oxydo-réduction est inférieur à -0,94V (e.c.s.) en milieu aqueux ou hydroalcoolique à une température comprise entre -10 et 60°C, soit par voie électrochimique en solution acide aqueuse ou hydroalcoolique contenant jusqu'à 50% d'alcool à une température comprise entre 0 et 60°C, sous agitation constante et sous atmosphère d'azote, dans une cellule d'électrolyse dans laquelle la cathode est constituée d'une nappe de mercure et le potentiel de l'électrode de travail est compris entre -0,9 et -1,1 V (e.c.s.),
puis transforme éventuellement le produit obtenu en un sel d'addition avec un acide.

## Claims

1. Process for preparing a streptogramin derivative of general formula: 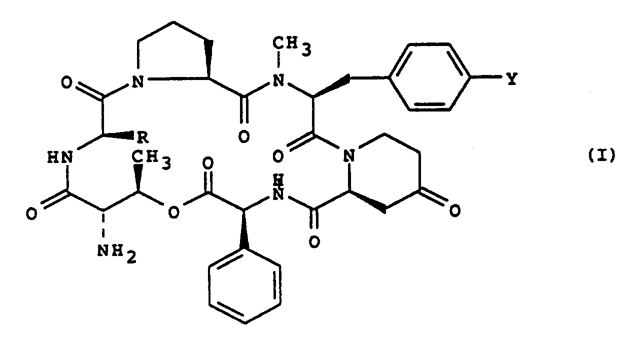 in which Y is a hydrogen atom or a methylamino or dimethylamino radical, or a radical of structure: 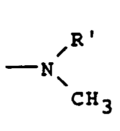 in which R' is an amino-protecting radical and R is a methyl or ethyl radical, characterized in that the reductive cleavage of a streptogramin derivative of general formula: 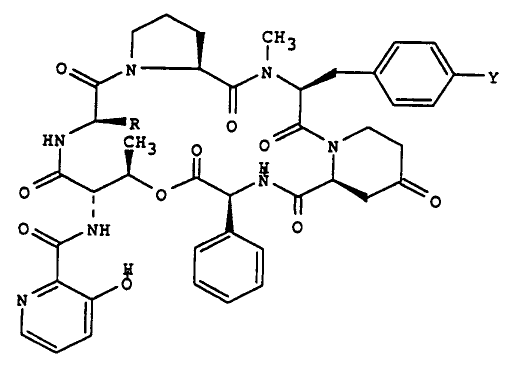 in which Y and R are defined as above, is performed in an acidic medium,
either with a strong acid at a pH below 2, in the presence of a reducing metal whose redox potential is less than -0.94 V (s.c.e.) in an aqueous or aqueous-alcoholic medium at a temperature of between -10 and 60°C, or electrochemically in aqueous acidic solution or aqueous-alcoholic acidic solution containing up to 50 % of alcohol at a temperature of between 0 and 60°C, with constant stirring and under a nitrogen atmosphere, in an electrolysis cell in which the cathode consists of a bed of mercury and the potential of the working electrode is between -0.9 and -1.1 V (s.c.e.),
and the product obtained is then optionally converted to an addition salt with an acid.

## Patentansprüche

1. Verfahren zur Herstellung eines Streptograminderivats der allgemeinen Formel 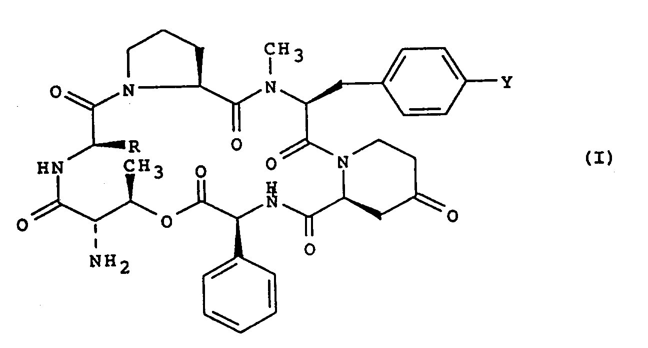 worin Y für ein Wasserstoffatom oder einen Methylamino- oder Dimethylaminorest oder einen Rest der Struktur 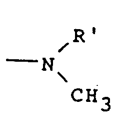 steht, in der R' eine Aminoschutzgruppe darstellt und R für einen Methyl- oder Ethylrest steht, dadurch gekennzeichnet, daß man eine reduktive Spaltung in saurem Milieu eines Streptograminderivats der allgemeinen Formel 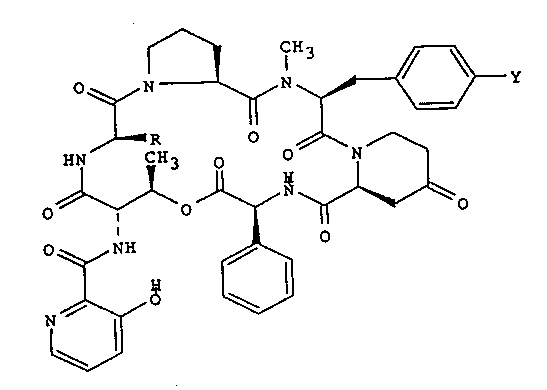 worin Y und R wie vorstehend definiert sind, entweder mit einer starken Säure bei einem pH von niedriger als 2 in Gegenwart eines reduzierenden Metalls, dessen Redoxpotential geringer als -0,94 V [e.c.s. (Standard: Kalomelelektrode mit gesättigter Kaliumchloridlösung)] ist, in wäßrigem oder wäßrig-alkoholischem Milieu bei einer Temperatur zwischen -10 und 60°C oder auf elektrochemischem Weg in wäßriger saurer Lösung oder in wäßrig-alkoholischer Lösung, enthaltend bis zu 50 % Alkohol, bei einer Temperatur zwischen 0 und 60°C unter konstantem Rühren und unter Stickstoffatmosphäre in einer Elektrolysezelle vornimmt, in der die Kathode aus einem Quecksilbermantel besteht und das Potential der Arbeitselektrode zwischen -0,9 und -1,1 V (e.c.s.) liegt,
und hiernach gegebenenfalls das erhaltene Produkt in ein Additionssalz mit einer Säure überführt.
